# EUROPÄISCHE PATENTSCHRIFT

(11) **EP 1 603 868 B1**
(45) Veröffentlichungstag und Bekanntmachung des Hinweises auf die Patenterteilung: **13.09.2006**
(21) Anmeldenummer: 04713039.8
(22) Anmeldetag: 20.02.2004
(51) Int. Cl.: C07C 323/22, C07C 319/14

(54) **VERFAHREN ZUR HERSTELLUNG VON ALPHA-(3-ARYLTHIO)-ACETOPHENONEN**
METHOD FOR PRODUCING ALPHA-(3-ARYLTHIO)-ACETOPHENONES
PROCEDE DE PRODUCTION D' ALPHA-(3-ARYLTHIO)-ACETOPHENONES

(30) Priorität: 06.03.2003 DE 10309645
(43) Veröffentlichungstag der Anmeldung: 14.12.2005
(73) Patentinhaber: BASF Aktiengesellschaft, 67056 Ludwigshafen (DE)
(72) Erfinder: ALTMAYER, Marco, 68199 Ilvesheim (DE); SIEGEL, Wolfgang, 67117 Limburgerhof (DE)
(86) Internationale Anmeldenummer: PCT/EP2004/001676
(87) Internationale Veröffentlichungsnummer: WO 2004/078705

(56) Entgegenhaltungen:
- EP-A- 0 735 016
- WO-A-02/42261
- WO-A-03/010156
- FR-A- 2 329 270
- KIM S ET AL: "A FACILE SYNTHESIS OF 3-ARYL-SUBSTITUTED-BENZOTHIOPHENES VIA A LEWIS ACID MEDIATED CYCLIZATION OF 2-ARYLTHIO-ACETOPHENONES" TETRAHEDRON LETTERS, ELSEVIER SCIENCE PUBLISHERS, AMSTERDAM, NL, Bd. 40, 1999, Seiten 2909-2912, XP001068955 ISSN: 0040-4039
- JONES C D ET AL: "ANTIEGTROGENS. 21 STRUCTURE-ACTIVITY STUDIES IN A SERIES OF 3-AROYL-2-ARYLBENZOUBTHIOPHENE DERIVATIVES LEADING TO U6-HYDROXY-2-(4-HYDROXYPHENYL)BENZOUBTHIEN -3-YLU4-U2-1PEPERIDINYL)ETH OXYL-PHENYL METHANONE HYDROCHLORIDE (LY156758), A REMARKABLY EFFECTIVE ESTROGEN ANGANOSIST WITH ONLY MINIMAL INTRINS" JOURNAL OF MEDICINAL CHEMISTRY, AMERICAN CHEMICAL SOCIETY. WASHINGTON, US, Bd. 27, Nr. 8, 1. August 1984 (1984-08-01), Seiten 1057-1066, XP000560343 ISSN: 0022-2623
- DATABASE CROSSFIRE BEILSTEIN BEILSTEIN INSTITUT ZUR FOERDERUNG DER CHEMISCHEN WISSENSCHAFTEN, FRANKFURT AM MAIN, DE; Database-Accession no. 3760117 (REACTION ID) XP002288431 & OLIVATO, PAULO ROBERTO ET AL.: MAGN. RESON. CHEM., Bd. 25, 1987, Seiten 179-180,

## Beschreibung

Die vorliegende Erfindung betrifft ein verbessertes Verfahren zur Herstellung von α-(3-Arylthio)-acetophenonen der allgemeinen Formel 1 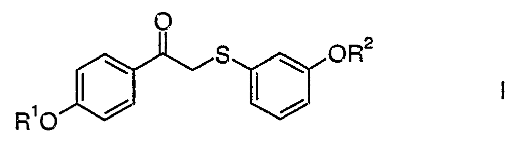 in der die Substituenten R¹ und R² unabhängig voneinander für C₁-C₆-Alkyl oder einen gegebenenfalls substituierten Phenyl- oder Benzylrest stehen.

Die Verbindungen der Formel I sind Zwischenprodukte bei der Synthese von pharmazeutisch aktiven Substanzen; 1-(4-Methoxyphenyl)-2-[(3-methoxyphenyl)thio]ethanon ist ein Baustein zur Herstellung des Anti-Osteoporose-Wirkstoffs Raloxifen.

Die Herstellung von Verbindungen der Formel I ist u. a. aus der WO 02/42261 bekannt. Nach dieser Lehre werden α-Chlor- oder α-Bromacetophenone aus dem entsprechenden Acetophenon und einem Halogenierungsmittel hergestellt und isoliert. Anschließend werden sie in einem mit Wasser nicht mischbaren Lösungsmittel mit dem entsprechenden Thiophenol in alkalischer wässriger Lösung umgesetzt, wobei das Halogenatom durch das Thiolatanion nucleophil substituiert wird.

Diese Verfahrensweise hat den Nachteil, dass die α-Chlor- oder α-Bromverbindungen isoliert werden müssen. Dies führt zum einem zu nicht unerheblichen Ausbeuteverlusten bei der Aufarbeitung der Reaktionsmischung sowie bei der nachfolgenden Wertprodukt-Reinigung. Zum anderen ist die Isolierung im technischen Maßstab aufwändig, da die Verbindungen stark tränenreizend sowie sensibilisierend sind und somit kontaminationsfrei abgefüllt werden müssen.

Es bestand daher die Aufgabe, ein Verfahren zur Herstellung von Verbindungen der allgemeinen Formel I zu finden, bei dem auf die Isolierung, Reinigung, Trocknung und Abfüllung von α-Chlor- bzw. α-Bromacetophenonen verzichtet werden kann.

Demgemäß wurde das eingangs beschriebene Verfahren gefunden, das dadurch gekennzeichnet ist, dass man
A) Acetophenone der allgemeinen Formel II, in der der Substituent R¹ die oben angegebene Bedeutung hat, 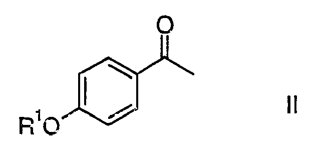 mit Sulfurylchlorid umsetzt und anschließend hydrolysiert wird, und
B) die so erhaltene Reaktionsmischung mit einem Thiophenol der allgemeinen Formel III, in der der Substituent R² die oben angegebene Bedeutung hat, umsetzt.

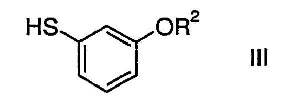

Das erfindungsgemäße Verfahren dient zur Herstellung von Verbindungen der Formel I, bevorzugt von 1-(4-Methoxyphenyl)-2-[(3-methoxyphenyl)thio]ethanon.

Als eine Ausgangsverbindung dienen Acetophenone der allgemeinen Formel II, in der der Substituent R¹ für C₁-C₆-Alkyl wie Methyl, Ethyl, iso-Propyl, n-Butyl oder iso-Butyl, Phenyl oder Benzyl steht, wobei die Phenyl- und Benzylreste unter den Reaktionsbedingungen inerte Substituenten wie zum Beispiel Halogen oder Oxyalkyl tragen können. Bevorzugt werden für R¹ kurzkettige Alkylreste, insbesondere Methyl.

Das Acetophenon der allgemeinen Formel II wird in Reaktionsschritt A) mit Sulfurylchlorid SO₂Cl₂ umgesetzt. Dabei wird in der Regel ein molarer Überschuß an Sulfurylchlorid eingesetzt, bevorzugt 1,1 bis 2 mol Sulfurylchlorid pro Mol Acetophenon, besonders bevorzugt 1,7 bis 1,8 mol pro Mol.

Die Chlorierung wird bevorzugt in Anwesenheit eines aliphatischen Alkohols durchgeführt. Bevorzugt sind gesättigte unverzweigte oder verzweigtkettige Alkohole, insbesondere solche mit ein bis zehn C-Atomen, besonders Methanol, Ethanol, 1-Propanol, 2-Propanol, 1-Butanol und 2-Butanol.
Die Alkohole können sowohl als Einzelsubstanz als auch als Gemisch in dem Verfahren eingesetzt werden.

Die aliphatischen Alkohole werden im allgemeinen in Mengen von 0,1 bis 10 Mol, bevorzugt von 2 Mol bis 6 Mol, pro Mol Acetophenon der allgemeinen Formel II eingesetzt.

Als zusätzliche Lösungsmittel können inerte organische Lösungsmittel verwendet werden wie gesättigte aliphatische Kohlenwasserstoffe, beispielsweise Hexan, Heptan oder Octan, sowie cycloaliphatische Kohtenwasserstoffe wie Cyclohexan. Desweiteren können chlorierte aliphatische Kohlenwasserstoffe, z. B. Methylenchlorid, Chloroform, Tetrachlormethan verwendet werden. Auch aromatische Lösungsmittel sind einsetzbar wie Benzol, Toluol, Ethylbenzol, Xylole wie o-Xylol, Halogenaromaten wie Chlorbenzol und Dichlorbenzole.

Bevorzugt wird ein Lösungmittelgemisch aus einem aromatischen Lösungsmittel, bevorzugt Toluol, und einem aliphatischen Alkohol wie Methanol, Ethanol, 2-Propanol und 1-Butanol, bevorzugt 1-Butanol. Vorteilhaft ist ein gewichtsmäßiger Überschuß des Alkohols, wobei Mischungsverhältnisse von 1 Gewichtsteil des aromatischen Lösungsmittels mit 2 bis 20, bevorzugt 8 bis 12 Gewichtsteilen des aliphatischen Alkohols sich besonders bewährt haben.

In diesem Lösungsmittel oder Lösungsmittelgemisch wird das Acetophenon der allgemeinen Formel II üblicherweise vorgelegt und Sulfurylchlorid wird zugesetzt. Die Umsetzung kann beispielsweise in einem Rührkessel vorgenommen werden. Die Reaktion kann bei Normaldruck und einer Temperatur von 0 bis 50°C durchgeführt werden; bei höheren Temperaturen verringert sich die Selektivität der Reaktion, niedrigere Temperaturen liefern keine signifikanten Vorteile hinsichtlich Selektivität, Ausbeute und Verfahrenstechnik.

Nach der Zugabe des Sulfurylchlorids kann noch eine Weile nachgerührt werden. Das Reaktionsgemisch wird anschließend hydrolysiert, bis überschüssiges Sulfurylchlorid abreagiert ist. In einer bevorzugten Ausführungsvariante wird nach der Hydrolyse die Mischung schwach sauer gestellt, der pH-Wert auf 5,0 bis 6,0 eingestellt. Dies geschieht durch Zugabe von Basen wie Natriumcarbonat, Calciumhydroxid, Kaliumhydroxid oder bevorzugt Natriumhydroxid.

Im Reaktionsschritt B) wird das gemäß Reaktionsschritt A) in situ hergestellte α-Chloracetophenon mit einem Thiophenol der Formel III umgesetzt, in der der Substituent R² für C₁-C₆-Alkyl wie Methyl, Ethyl, iso-Propyl, n-Butyl oder iso-Butyl, Phenyl oder Benzyl steht, wobei die Phenyl- und Benzylreste unter den Reaktionsbedingungen inerte Substituenten wie zum Beispiel Halogen oder Oxyalkyl tragen können. Bevorzugt werden für R² kurzkettige Alkylreste, insbesondere Methyl.

Das Thiophenol der Formel III wird der Reaktionsmischung aus Reaktionsschritt A) zugesetzt. Die Menge an Thiophenol beträgt im allgemeinen 0,8 bis 2,0 mol Thiophenol pro Mol Acetophenon der Formel II, bevorzugt 0,90 bis 1,05 mol pro Mol.

In einer bevorzugten Ausführungsform wird der pH-Wert nach der Zugabe des Thiophenols der allgemeinen Formel III auf 6,0 bis 9,0 eingestellt, wozu Basen wie Natriumcarbonat, Calciumhydroxid, Kaliumhydroxid oder bevorzugt Natriumhydroxid verwendet werden können.

Das Ende der Reaktion kann mittels Gaschromatographie überprüft werden.

Das Reaktionsprodukt kann in an sich bekannter Weise isoliert werden. Dazu kühlt man den Reaktionsansatz bevorzugt auf 0 bis 5°C ab, impft mit Produktkristallen an und rührt ca. 30 Minuten nach. Das Produkt wird filtriert mit Wasser und anschließend mit Methanol digeriert, gewaschen und getrocknet.

Das erfindungsgemäße Verfahren erlaubt die Herstellung von α-(3-Arylthio)-acetophenonen der allgemeinen Formel I in guter Ausbeute mit hoher Reinheit. Dabei werden die Nachteile des Standes der Technik in verfahrenstechnisch unerwartet einfacher Weise vermieden und insbesondere lassen sich die aufwändigen Maßnahmen zur Verhinderung von Kontakt mit den Zwischenprodukten erheblich reduzieren.

### Beispiel 1 - Herstellung von 1-(4-Methoxyphenyl)-2-[(3-methoxyphenyl)thio]ethanon

48,1 g (0,32 mol) 4-Methoxyacetophenon in 11 ml Toluol und 117 ml 1-Butanol wurden vorgelegt. Unter Eiskühlung wurden bei 25 - 30°C 74,62 g (0,55 mol) Sulfurylchlorid (Molverhältnis Acetophenon:Sulfurylchlorid 1:1,7) zugetropft. Nach 30 Minuten Nachrühren wurde die Reaktionsmischung mit 210 ml Wasser hydrolysiert und mit 40 ml konz. Natronlauge auf einen pH-Wert von 6,0 eingestellt, wobei die Temperatur 30°C nicht überstieg.

Dann wurden 44,80 g (0.32 mol) 3-Methoxythiophenol zugegeben und der pH-Wert mit konz. Natronlauge auf 8,5 eingestellt. Nach 1 h Nachrühren wurde auf 0 - 5°C abgekühlt, mit Produktkristallen angeimpft und 30 Minuten nachgerührt. Die Kristalle wurden abfiltriert, mit 200 ml Wasser digeriert, dann mit 200 ml Wasser gewaschen, mit 75 ml Methanol digeriert und mit 75 ml Methanol gewaschen. Die Kristalle wurden bei 30°C getrocknet.

Ausbeute: 64,8 g (0,225 mol) 1-(4-Methoxyphenyl)-2-[(3-methoxyphenyl)thio]ethanon, 70%
Reinheit (GC): 99,5 %

### Beispiel 2 - Herstellung von 1-(4-Methoxyphenyl)-2-[(3-methoxyphenyl)thio]ethanon

48,1 g (0,32 mol) 4-Methoxyacetophenon in 11 ml Toluol und 117 ml 1-Butanol wurden vorgelegt. Unter Eiskühlung wurden bei 25 - 30°C 74,62 g (0,55 mol) Sulfurylchlorid (Molverhältnis Acetophenon:Sulfurylchlorid 1:1,7) zugetropft. Nach 30 Minuten Nachrühren wurde die Reaktionsmischung mit 210 ml Wasser hydrolysiert und mit 40 ml konz. Natronlauge auf einen pH-Wert von 6,0 eingestellt, wobei die Temperatur 30°C nicht überstieg.

Dann wurden 44,80 g (0.32 mol) 3-Methoxythiophenol zugegeben und der pH-Wert mit konz. Natronlauge auf 7,0 eingestellt. Nach 1 h Nachrühren wurde auf 0 - 5°C abgekühlt, mit Produktkristallen angeimpft und 30 Minuten nachgerührt. Die Kristalle wurden abfiltriert, mit 200 ml Wasser digeriert, dann mit 200 ml Wasser gewaschen, mit 75 ml Methanol digeriert und mit 75 ml Methanol gewaschen. Die Kristalle wurden bei 30°C getrocknet.

Ausbeute: 63,6 g (0,221 mol) 1-(4-Methoxyphenyl)-2-[(3-methoxyphenyl)thio]ethanon, 69%

Reinheit (GC): 97,5 %

## Patentansprüche

1. Verfahren zur Herstellung von α-(3-Arylthio)-acetophenonen der allgemeinen Formel I 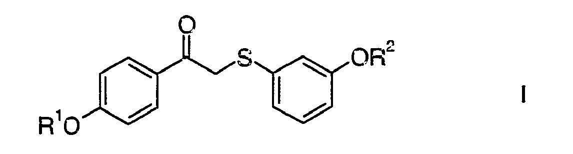 in der die Substituenten R¹ und R² unabhängig voneinander für C₁-C₆-Alkyl oder einen gegebenenfalls substituierten Phenyl- oder Benzylrest stehen, **dadurch gekennzeichnet, dass** man
A) Acetophenone der allgemeinen Formel II, in der der Substituent R¹ die oben angegebene Bedeutung hat, 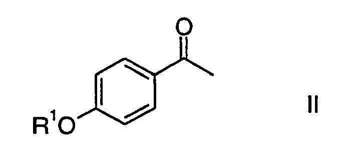 mit Sulfurylchlorid umsetzt und anschließend hydrolysiert, und
B) die so erhaltene Reaktionsmischung mit einem Thiophenol der allgemeinen Formel III, in der der Substituent R² die oben angegebene Bedeutung hat, umsetzt.

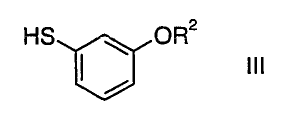

2. Verfahren nach Anspruch 1, **dadurch gekennzeichnet, dass** man 1-(4-Methoxyphenyl)-2-[(3-methoxyphenyl)thio]ethanon herstellt.

3. Verfahren nach Anspruch 1 oder 2, **dadurch gekennzeichnet, dass** man Reaktionsschritt A) in einem Lösungsmittelgemisch aus einem aromatischen Lösungsmittel und einem aliphatischen Alkohol vornimmt.

4. Verfahren nach den Ansprüchen 1 bis 3, **dadurch gekennzeichnet, dass** man nach der Hydrolyse und vor Zugabe des Thiophenols der allgemeinen Formel III einen pH-Wert von 5,0 bis 6,0 einstellt.

5. Verfahren nach den Ansprüchen 1 bis 4, **dadurch gekennzeichnet, dass** man Reaktionsschritt B) bei einem pH-Wert von 6,0 bis 9,0 durchführt.

## Claims

1. A process for preparing *α*-(3-arylthio)acetophenones of the general formula I 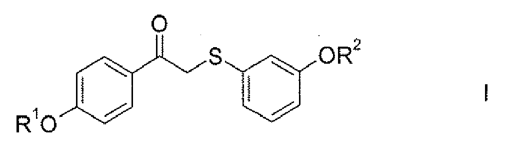 where the substituents R¹ and R² are each independently C₁-C₆-alkyl or an optionally substituted phenyl or benzyl radical, which comprises
A) reacting acetophenones of the general formula II where the substituent R¹ is as defined above 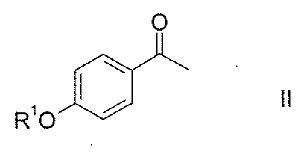 with sulfuryl chloride and subsequently hydrolyzing, and
B) reacting the reaction mixture obtained in this way with a thiophenol of the general formula III where the substituent R² is as defined above

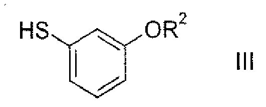

2. The process according to claim 1, wherein 1-(4-methoxyphenyl)-2-[(3-methoxyphenyl)thio]ethanone is prepared.

3. The process according to claim 1 or 2, wherein reaction step A) is carried out in a solvent mixture of an aromatic solvent and an aliphatic alcohol.

4. The process according to any of claims 1 to 3, wherein the pH is adjusted to from 5.0 to 6.0 after the hydrolysis and before the addition of the thiophenol of the general formula III.

5. The process according to any of claims 1 to 4, wherein reaction step B) is carried out at a pH of from 6.0 to 9.0.

## Revendications

1. Procédé de fabrication de α-(3-arylthio)-acétophénones de formule générale I : 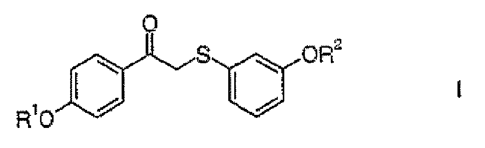 dans laquelle les substituants R¹ et R² représentent, indépendamment l'un de l'autre, un groupe alkyle en C₁₋C₆ ou un radical phényle ou benzyle éventuellement substitué, **caractérisé en ce que** l'on fait réagir
A) des acétophénones de formule générale II, dans laquelle le substituant R¹ a la signification indiquée ci-dessus : 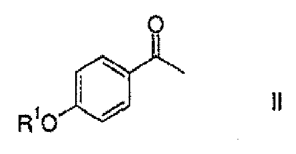 avec du chlorure de sulfuryle et l'on en fait ensuite l'hydrolyse, et
B) l'on fait réagir le mélange réactionnel ainsi obtenu avec un thiophénol de formule générale III, dans laquelle le substituant R² a la signification indiquée ci-dessus.

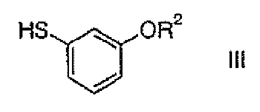

2. Procédé selon la revendication 1, **caractérisé en ce que** l'on fabrique de la 1-(4-méthoxy-phényl)-2-[(3-méthoxyphényl) thio] éthanone.

3. Procédé selon la revendication 1 ou 2, **caractérisé en ce que** l'on effectue l'étape réactionnelle A) dans un mélange d'un solvant aromatique et d'un alcool aliphatique.

4. Procédé selon les revendications 1 à 3, **caractérisé en ce que** l'on ajuste une valeur du pH de 5,0 à 6,0 après l'hydrolyse et avant l'addition du thiophénol de formule générale III.

5. Procédé selon les revendications 1 à 4, **caractérisé en ce que** l'on effectue l'étape réactionnelle B) à une valeur du pH de 6,0 à 9,0.
